# EUROPEAN PATENT APPLICATION

(11) **EP 4 036 580 A1**
(43) Date of publication of application: **03.08.2022**
(21) Application number: 21386009.1
(22) Date of filing: 01.02.2021
(51) Int. Cl.: G01N 33/543, A61K 9/00, A61K 9/51, A61K 47/00

(54) **DRUG LOADED CAVITATION AGENT**

(71) Applicant: Oxford University Innovation Limited, Oxford, OX2 0JB (GB)
(72) Inventor: The designation of the inventor has not yet been filed
(74) Representative: J A Kemp LLP

(57) **Abstract**

The invention describes ultrasound-responsive particles having a core containing one or more therapeutic or diagnostic agents, and a polypeptide shell. The surface of the particle has one or more indentations which are generally able to entrap a gas bubble. The particles are capable of generating inertial cavitation on exposure to ultrasound. The inertial cavitation properties of the particles can be used to enhance delivery of the therapeutic or diagnostic agents to target sites in vivo.

## Description

### FIELD OF THE INVENTION

The present invention relates to ultrasound-responsive polypeptide particles containing one or more therapeutic or diagnostic agents. The particles are useful in delivering drugs or diagnostic agents to target sites in the body. They are also useful themselves in the treatment or prevention of disease.

### BACKGROUND

Ultrasound-responsive particles have been used for some time to assist delivery of therapeutic or diagnostic agents to the body. For instance, drug can be co-administered with microbubbles, which act as cavitation nuclei. The action of ultrasound on the microbubbles leads to a short burst of microstreaming caused by the cavitation effects. This can increase the delivery of the drug at the target site. Administration of ultrasound can also improve the permeability of physiological barriers such as the vasculature walls, and thereby increase drug delivery.

However, microbubbles provide only a very short duration of action, with cavitation lasting for no more than about 30 seconds. Moreover, the bubbles themselves are too large to exit the vasculature, which limits their effectiveness in delivery to the extravascular space.

Nanoparticulate cavitation agents have also been described (WO 2015/075422) which have the benefit of longer duration of action and are small enough to pass into the extravascular space. The particles described are formed by seed polymerisation to generate cup-shaped plastic particles, capable of holding a gas bubble in the cavity of the cup. These particles have been found to disseminate throughout a tumour mass, for example, on exposure to ultrasound for 30 seconds. However, the particles described are plastic and are non-biodegradable.

There is therefore a need for new ultrasound-responsive particles and/or new methods for ultrasound-responsive delivery of drugs and diagnostic agents to a target site, which address the problems discussed above.

### SUMMARY OF THE INVENTION

The present inventors have found that biodegradable, ultrasound-responsive particles can be made from polypeptides (e.g. proteins). The particles generate inertial cavitation on response to ultrasound and can be made at submicron sizes. The particles have a good duration of action, having been found to generate cavitation for a period of around 10 minutes following ultrasound exposure. If desired, they can be manufactured at a size which is small enough to pass through the walls of the vasculature. Therefore, the particles themselves, and any therapeutic or diagnostic agent which they contain, can be effectively delivered to target sites outside of the vasculature (e.g. to tumours). The particles are also highly stable. The particles therefore provide an effective means of generating inertial cavitation in a therapeutic or diagnostic setting, and thereby delivering their payload of therapeutic or diagnostic agent to the target site in a highly selective manner.

The present invention therefore provides, in a first aspect (1):
1. A polypeptide particle for inducing cavitation on exposure to ultrasound, the particle comprising:
   - a core comprising one or more therapeutic or diagnostic agents, and optionally a water-immiscible liquid; and
   - a polypeptide shell;
   wherein the particle has one or more surface indentations.

A particular advantage of the particles of the invention is that they can be effectively administered transdermally. Application of the particles of the invention to the skin, followed by applying ultrasound, generates an inertial cavitation effect sufficient to deliver the particles, together with any contained therapeutic or diagnostic agent, transdermally. The particles are therefore useful as a means to deliver agent transdermally, in particular in the treatment of skin diseases and disorders.

Also provided are the following aspects:
2. A polypeptide particle according to aspect 1, wherein the polypeptide shell comprises one or more non-immunomodulatory polypeptides, preferably the polypeptide shell comprises at least 95% by weight non-immunomodulatory polypeptides.
3. A polypeptide particle according to aspect 1 or 2, wherein the polypeptide shell consists essentially of one or more human blood proteins, preferably it is a human serum albumin shell.
4. A polypeptide particle according to any one of the preceding aspects, wherein the particle size is in the range of from 100nm to 1000nm, preferably from 200nm to 700nm.
5. A polypeptide particle according to any one of the preceding aspects, wherein the cross section of at least one surface indentation forms a conic section.
6. A polypeptide particle according to any one of the preceding aspects, wherein at least one surface indentation has a depth of at least 10nm, and/or an opening size of at least 50nm.
7. A polypeptide particle according to any one of the preceding aspects, wherein the particle comprises one or two indentations having an opening size which is 20% or more of the size of the particle.
8. A polypeptide particle according to any one of the preceding aspects, wherein the polypeptide shell is conjugated to one or more ligands selected from a peptide, a protein, a sugar, a nanoparticle and a nucleic acid.
9. A polypeptide particle according to any one of the preceding aspects, which particle is capable of generating inertial cavitation when the particle is suspended in water and subject to ultrasound at 1.2MPa and 265kHz, preferably the particle is capable of generating inertial cavitation when the particle is suspended in water and subject to ultrasound at IMPa and 500kHz, more preferably the particle is 450nm or less in size and is capable of generating inertial cavitation when the particle is suspended in water and subject to ultrasound at 1.5 MPa and 500kHz.
10. A polypeptide particle according to any one of the preceding aspects, wherein the core comprises one or more therapeutic agents selected from small-molecule drugs, including chemotherapy agents, anti-thrombotic drugs, anti-inflammatory drugs, steroids, cardiovascular drugs, and therapeutic agents useful in the treatment of cancer, skin conditions, diseases or disorders of the brain, and therapeutic agents useful in pain management.
11. A polypeptide particle according to any one of the preceding aspects, wherein the core is a water-immiscible liquid core comprising a water-immiscible liquid and one or more therapeutic or diagnostic agents.
12. A composition comprising one or more polypeptide particles according to any one of the preceding aspects, together with one or more pharmaceutically acceptable carriers or diluents.
13. A method of producing a polypeptide particle according to any one of aspects 1 to 11, comprising:
   - providing a water-immiscible phase comprising one or more volatile components, one or more therapeutic or diagnostic agents, and optionally one or more non-volatile components;
   - mixing said water-immiscible phase with an aqueous solution of at least one polypeptide comprising at least two cysteine residues;
   - cross-linking the cysteine residues to generate a particle having a core comprising the water-immiscible phase and a shell comprising the at least one polypeptide; and
   - creating one or more indentations in the particle by subjecting the particle to reduced pressure conditions, to remove volatile component from the core.
14. A method according to aspect 13, wherein the ratio of volatile component to non-volatile component in the water-immiscible phase is from 1:20 to 20:1.
15. A method according to aspect 13 or 14, which further comprises:
   - drying the particle; and
   - optionally re-suspending the particle in a liquid medium.
16. A method according to any one of aspects 13 to 15, which further comprises:
   - lyophilising the particle; and
   - optionally re-suspending the particle in a liquid medium;
   wherein preferably the lyophilisation and optional resuspension are carried out after drying the particle and re-suspending the particle in a liquid medium.
17. A method according to any one of aspects 13 to 16, which further comprises a step of purification of the particles.
18. A polypeptide particle according to any one of aspects 1 to 11, or a composition according to claim 12, for use in a method of diagnosis or therapy.
19. A polypeptide particle or composition for use according to aspect 18, wherein the method comprises administering the polypeptide particle or composition, applying ultrasound and inducing inertial cavitation.
20. A polypeptide particle or composition for use according to aspect 18 or 19, wherein ultrasound is delivered at a frequency of from 100 to 3000kHz and a peak negative pressure of from 0.5 to 7.0 MPa; preferably wherein (a) the polypeptide particle or composition is administered transdermally and ultrasound is delivered at a frequency of from 100 to 500 kHz and a peak negative pressure of from 0.5 MPa to 2.0 MPa; or (b) the polypeptide particle or composition is administered parenterally, preferably by injection, and ultrasound is delivered at a frequency of from 200 to 3000 kHz and a peak negative pressure of from 1.0 MPa to 7.0 MPa.
21. A polypeptide particle or composition for use according to any one of aspects 18 to 20, wherein the method is for delivery of the therapeutic or diagnostic agent to a target site in vivo, preferably wherein the polypeptide particle or composition is for use in the delivery of the therapeutic or diagnostic agent to a tumour, to the heart, to the brain or to the skin.
22. A polypeptide particle or composition for use according to any one of aspects 18 to 21, wherein the polypeptide particle or composition is administered parenterally, preferably wherein the polypeptide particle or composition is administered by intravenous, intramuscular, intradermal or subcutaneous administration.
23. A polypeptide particle or composition for use according to any one of aspects 18 to 21, wherein the polypeptide particle or composition is administered by transdermal administration.
24. A polypeptide particle or composition for use according to any one of aspects 18 to 23, for use in treating or preventing tumour, cardiovascular disease, infection or a neurological disease or disorder.
25. A polypeptide particle or composition for use according to any one of aspects 18 to 23, for use in treating or preventing a disease or condition of the skin.
26. Use of a polypeptide particle according to any one of aspects 1 to 11, or a composition according to aspect 12, in the manufacture of a medicament for use in a method of diagnosis or therapy, in particular wherein the method is as set out in any one of aspects 19 to 23.
27. Use according to aspect 26, wherein the medicament is for use in treating or preventing tumour, cardiovascular disease, infection or a neurological disease or disorder.
28. Use according to aspect 26, wherein the medicament is for use in treating or preventing a disease or condition of the skin.
29. A method of treatment or diagnosis of a subject, which method comprises administering to the subject an effective amount of a polypeptide particle according to any one of aspects 1 to 11, or a composition according to aspect 12, in particular wherein the method is as set out in any one of aspects 18 to 23.
30. A method of treating, ameliorating, preventing or reducing the incidence of tumour, cardiovascular disease, infection or a neurological disease or disorder in a subject, which method comprises administering to the subject an effective amount of a polypeptide particle according to any one of aspects 1 to 11, or a composition according to aspect 12, in particular wherein the method is as set out in any one of aspects 19 to 23.
31. A method of treating or preventing a disease or condition of the skin in a subject, which method comprises administering to the subject an effective amount of a polypeptide particle according to any one of aspects 1 to 11, or a composition according to aspect 12, in particular wherein the method is as set out in any one of aspects 19 to 23.

### BRIEF DESCRIPTION OF THE FIGURES

Figure 1 is a schematic representation of a particle according to the invention having a single indentation. S represents the particle size and d represents the depth of the indentation. P represents the size of the opening of the indentation, also referred to as opening diameter.
Figure 2 provides a schematic representation of the process used to make the ultrasound-responsive particles of the invention, as well as a process for making substantially spherical particles.
Figures 3a and b show 3D reconstruction of Z-stacks generated via spinning disc confocal microscopy with particles pre-conjugated with AlexaFluor 488 NHS (Fig 3a) and CY5 NHS (Fig 3b); Figure 3c shows scanning electron microscopy (SEM) images of particles according to the invention; Figure 3d shows a light microscopy image of a particle according to the invention; Figure 3e shows UPLC analysis of a digestion of particles of the invention having a terazonsin core (top panel) and a reference for terazonsin (bottom panel).
Figure 4 shows the average diameter (Z-average) of particles generated by varying the nature of the volatile component inside the core.
Figure 5 shows the average diameter (Z-average) of particles generated by varying the relative amount of the volatile component inside the core.
Figure 6 shows the effect of particle size on cavitation ability across four pressure settings.
Figure 7 shows the effect of percentage volatile component in core on cavitation ability across four pressure settings.
Figure 8 shows a representative energy trace demonstrating persistent inertial cavitation of particles produced according to the invention.
Figure 8A shows the energy detected by PCD over time at 1.7 MPa during in vivo transdermal delivery of particles of the invention and luciferase pDNA.
Figure 9 provides a schematic of the ultrasound hardware used in in vivo cavitation experiments
Figure 10 shows Day-7 ELISA comparing the immune response to BSA protein, following delivery of particles made from BSA both transdermally and via a range of injections.
Figure 11 shows bioluminescence measurements as determined via IVIS imaging, following administration of DNA encoding luciferase, which may be via transdermal administration using cavitation agents according to the invention, using alternative cavitation agents, or using alternative routes of administration.

### DETAILED DESCRIPTION OF THE INVENTION

### Definitions

As used herein, an ultrasound-responsive particle is a particle that produces a response, typically a cavitation response, when exposed to ultrasound. The particles of the invention which are suitable for inducing (i.e. capable of inducing, or adapted to induce) cavitation on exposure to ultrasound are ultrasound-responsive particles as described herein.

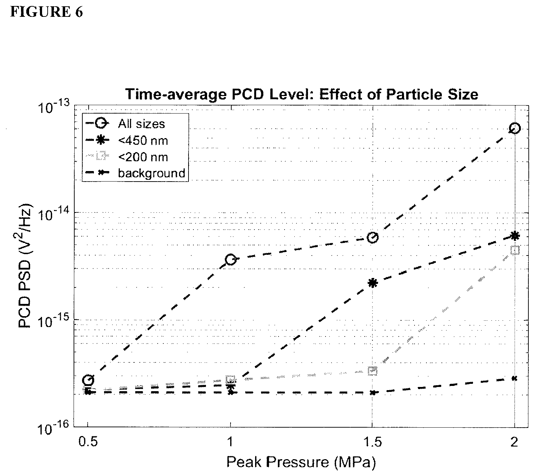

Cavitation occurs when a bubble in a fluid, e.g. a bubble associated with a particle of the invention, changes size or shape due to an external input, typically acoustic pressure. Typically, the particle generates an inertial cavitation response when exposed to ultrasound. Inertial cavitation is the effect which occurs when a bubble in a fluid, e.g. a bubble associated with a particle of the invention, grows following exposure to the acoustic pressure, and subsequently collapses. The bubble collapse may cause a shock wave and/or microstreaming in the surrounding fluid, and is accompanied by broadband (as opposed to tonal) acoustic emissions. Inertial cavitation may be detected by single- or multi-element detectors, including conventional hand held ultrasound arrays used for common medical diagnostics. Inertial cavitation may be assessed as the presence of broadband noise at a level clearly discernible from the background (e.g. at least 3 times the background root-mean-square noise) after excluding any tonal components. Particles are considered to generate an inertial cavitation response if broadband noise (e.g. measured as described above) is present on application of ultrasound (e.g. at 1.2MPa and 265kHz) to a suspension of particles in water. Preferably, the particles will generate an inertial cavitation response (i.e. broadband emission) when suspended in water and subject to ultrasound at IMPa and 500kHz. More preferably, the particles will generate an inertial cavitation response (i.e. broadband emission) when suspended in water and subject to ultrasound at 1.5MPa and 500kHz. Inertial cavitation is demonstrated, for example as shown in Figure 6 for particles having a range of sizes, by detection of passive cavitation (PCD) levels.

Particle size as defined herein is the maximum distance across a particle, i.e. the diameter in the case of a spherical particle. Although the particles defined herein are not necessarily spherical in shape, the particle size may be referred to herein as a particle diameter and both terms have the same meaning.

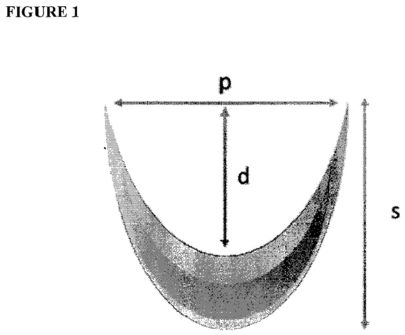

Particle size in the case of a composition containing a plurality of particles according to the invention is defined as the mean particle size. Mean particle size within a composition may be measured by a variety of techniques known to the skilled person (Malvern Nanosight, Beckman Coulter particle size, dynamic light scattering (DLS)). Typically the mean particle size is defined as the hydrodynamic diameter, as measured by dynamic light scattering (DLS). Particle size of an individual nanoparticle (s in Figure 1) may be measured by scanning electron microscopy (SEM).

As used herein, an indentation is a surface feature causing a cavity or depression to arise in the surface of the particle. Indentations are also referred to herein as surface indentations. Typically, an indentation is capable of entrapping or holding a gas bubble. The indentation has a depth depicted as d in Figure 1, which can be determined by microscopy (e.g. SEM or transmission microscopy, TEM). The depth of any indentation is typically at least about 10nm, preferably at least about 20nm, more preferably at least about 50nm. An indentation has an opening, which in the case of a part spherical indentation is the diameter of the indentation at the surface of the particle. The opening is depicted as p in Figure 1. The size of the opening is referred to herein as the opening size or the opening diameter, although the opening may or may not be circular in shape.

The shell thickness is the average thickness of the polypeptide shell and can be determined by SEM or TEM.

As used herein, the term "polypeptide" refers to a full-length protein, a portion of a protein, or a peptide characterized as a string of amino acids. Typically a polypeptide comprises at least 25, or 30, or 35, or 40, or 45, or 50 or 55 or 60 amino acids. For example, a polypeptide may contain 100 or more amino acids. As used herein, the term "protein" refers to a full length protein or a fragment of a protein.

The terms "fragment", "fragment of a protein" or "fragment of a polypeptide" as used herein refer to a string of amino acids or an amino acid sequence typically of reduced length relative to the or a reference protein or polypeptide and comprising, over the common portion, an amino acid sequence identical to the reference protein or polypeptide. In some cases the fragments referred to herein may be between 8 or 9 and 20, or 25, or 30, or 35, or 40, or 45, or 50 amino acids.

The term volatile as used herein indicates that a substance has a vapour pressure at 25°C greater than that of water (which has a vapour pressure about 3kPa) at the same temperature. Typically, a volatile substance, or a volatile component, is capable of vaporising at room temperature (25°C). A volatile component as used herein is thus a liquid, typically an organic solvent, which has a vapour pressure of at least 3 kPa, preferably at least 3.5 kPa, for example at least 5 kPa, at least 10 kPa or at least 15 kPa at 25°C.

Non-volatile indicates that a substance does not or substantially does not vaporise at room temperature (25°C). Non-volatile components are thus typically liquids, e.g. oils, which have a vapour pressure at 25°C which is no more than that of water (which has a vapour pressure about 3kPa) at the same temperature. A non-volatile component as used herein typically has a vapour pressure at 25°C of less than 3 kPa, preferably less than 2.5kPa, e.g. less than 2 kPa.

As described herein, the particles are useful in methods of therapy or diagnosis of a subject. The subject is a human or animal subject. In one aspect, the subject to be treated is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters.

The term "treatment" as used herein includes therapeutic and prophylactic treatment. Administration is typically in a "prophylactically effective amount" or a "therapeutically effective amount" (as the case may be, although prophylaxis may be considered therapy), this being sufficient to result in a clinical response or to show clinical benefit to the individual, e.g. an effective amount to prevent or delay onset of the disease or condition, to ameliorate one or more symptoms, to induce or prolong remission, or to delay relapse or recurrence.

An immunomodulatory polypeptide (or immunomodulatory protein) is a polypeptide (or protein) which induces an immune response on administration to a subject. Typically, the subject is a human and the protein is one which induces an immune response on administration to a human subject. Typically, administration of an immunomodulatory polypeptide (or immunomodulatory protein) causes the subject to raise antibodies against the polypeptide (or protein), although alternative immune responses may arise as well as or instead of raising antibodies.

A non-immunomodulatory polypeptide (or non-immunomodulatory protein) is a polypeptide (or protein) which does not give rise to an immune response on administration. Typically, such a polypeptide or protein is one which occurs naturally within the subject. An example of a non-immunomodulatory protein in the case of a human subject is human serum albumin. More generally, human blood proteins are examples of non-immunomodulatory proteins, including insulin, globulin and haemoglobin.

### Ultrasound-responsive polypeptide particles

The polypeptide particles of the invention are ultrasound-responsive particles. The particles are capable of generating a cavitation response when exposed to ultrasound in the presence of a fluid. Typically they generate inertial cavitation. Therefore, when the particles of the invention are present in a fluid, for example formulated into a gel or lotion, or present within the body, exposure to ultrasound will lead to inertial cavitation within that fluid.

The particles have one or more indentations (i.e. surface depressions, or cavities), which provides their ultrasound-responsive properties. Without being limiting on the invention, it is currently understood that the indentations or cavities in the particle surface are able to trap or hold a gas bubble. On exposure to ultrasound, the gas bubble grows and then collapses, causing an inertial cavitation effect. Thus, the particles have one or more indentations capable of causing inertial cavitation on exposure to ultrasound. In one aspect, the invention provides particles as described herein comprising a gas bubble entrapped at an indentation on the particle. The gas bubble may comprise air or oxygen, for example.

Typically, the particles are capable of causing inertial cavitation when suspended in water and subject to ultrasound at 1.2MPa and 265kHz. As described above, the ability of a particle to cause inertial cavitation can be assessed as the presence of broadband noise in the emitted signal. Preferably, the particles are capable of generating inertial cavitation when the particle is suspended in water and subject to ultrasound at 1MPa and 500kHz. More preferably, the particles are 450nm or less in size and are capable of generating inertial cavitation when the particles are suspended in water and subject to ultrasound at 1.5 MPa and 500kHz.

The particles typically have an average size of 8000nm or less, e.g. 5000nm or less, preferably they are nanoparticles having a particle size of 1000nm or less. Preferably, the particles have a size of from 100nm to 5000nm, preferably from 100nm to 1000nm. For example, the particles may have a size of at least 200nm, preferably from 200nm to 700nm, for example around 500nm. In one aspect, the particles have a size which is 450nm or less. Preferably, the mean particle size of a composition comprising one or more particles of the invention is from 100nm to 8000nm, e.g. from 100nm to 5000nm, preferably from 100nm to 1000nm, more preferably at least 200nm, preferably from 200nm to 700nm, for example around 500nm. In one aspect, the composition contains particles having a mean particle size which is 450nm or less. Particle size can be controlled, for example, by size filtration of particles.

In one aspect, the particle size may be from 100nm to 600nm, preferably from 100nm to 500nm, e.g. from 300nm to 500nm, for example from 100nm to 450nm or 300nm to 450nm. Particles of this size are small enough to pass through the walls of the vasculature and can therefore accumulate at desired sites outside the bloodstream. In the case of a composition, the mean particle size may be 1000nm or less, in particular from 100nm to 600nm, preferably from 100nm to 500nm, e.g. from 300nm to 500nm for example from 100nm to 450nm or 300nm to 450nm.

In an alternative aspect, the particles have a particle size of more than 500nm, preferably more than 600nm, for example from 600nm to 8000nm, e.g. from 600nm to 5000nm or from 700nm to 1000nm. In the case of a composition, the mean particle size may be more than 500nm, preferably more than 600nm, for example from 600nm to 5000nm or from 700nm to 1000nm.

Typically, the particles comprise a single indentation or comprise two indentations (in the latter case they are typically approximately toroid shaped, comprising two indentations, on opposite sides of the particle). Further smaller particle features, e.g. surface roughness wherein features have an opening size of 50nm or less, preferably 20nm or less, more preferably 10nm or less, may also be present. Typically, however, the particle has one or two indentations having an opening size of 50nm or greater.

The form of the indentation(s) may vary, as long as the indented particle is capable of causing inertial cavitation (e.g. the indentation(s) are capable of entrapping or holding a gas bubble within their cavity). Typically, an indentation capable of causing inertial cavitation has an opening size of at least 50nm or at least 100nm. The opening is, for example from 10 to 500nm, e.g. from 50 to 300nm or from 100 to 300nm. The opening size of an indentation as referred to herein relates to the diameter of the opening at the surface of the particle, in the case of a circular opening. In the case of a cavity having a non-circular cross-section at the opening, the opening size is the maximum dimension across the opening at the surface of the particle.

The size of the opening of an indentation may vary dependent on the size of the particle. For instance, the opening of an indentation may have a size (a diameter) which is at least 10% of the size (diameter) of the particle, e.g. at least 20% of the size (diameter) of the particle, preferably at least 40% of the size (diameter) of the particle. The opening size (diameter) of an indentation or cavity in the particle (its absolute size or relative size compared to the particle size) may be determined by imaging, e.g. SEM or AFM.

Typically, an indentation capable of causing inertial cavitation has a depth of at least 10nm, e.g. at least 50nm or at least 100nm. For example, the one or more indentations may have a depth of from 10nm to 250nm. The depth of the one or more indentations may be, for example, at least 10% of the particle size, for example at least 20% or at least 25% of the particle size. The depth of an indentation or cavity in the particle (its absolute size or relative size compared to the particle size) may be determined by imaging, e.g. SEM or AFM.

Typically, the cross-section of at least one indentation forms a conic section.

In one embodiment, the particle has a core comprising a water-immiscible phase comprising one or a mixture of non-volatile, water-immiscible components, (hereinafter a "water-immiscible core"). The content of non-volatile component (also referred to as water-immiscible liquid) as a proportion of the particle may vary widely. For example, the particle may comprise from 0.1 to 70% by weight non-volatile component and from 30 to 99.9% by weight polypeptide shell. The non-volatile component is typically an oil. The nature of the non-volatile component is not particularly limited and any biocompatible, pharmaceutically acceptable oil or mixture thereof can be used. Suitable components include sunflower oil, olive oil, soybean oil, coconut oil, safflower oil, cotton seed oil, sesame oil, orange oil, limonene oil, polyethylene glycols, or other non-volatile organic solvents and combinations of two or more of these oils. Sunflower oil, olive oil or a combination of these oils is preferred. The biocompatible, pharmaceutically acceptable oil is typically not itself pharmaceutically active.

In one embodiment, the core of the particle does not comprise water-immiscible liquid(s), or water-immiscible liquid is present only in a small amount, for example in an amount sufficient to stabilise or solubilise any therapeutic or diagnostic component. Typically, in this embodiment, the water-immiscible liquid is present in an amount of no more than 10 wt%, e.g. no more than 5 wt% compared to the total weight of the particle. In this embodiment, the core of the particle comprises one or more therapeutic or diagnostic components, which may be present in the core in either solid or liquid form, or they may be dissolved in, or mixed with, a small amount of a water-immiscible liquid (e.g. up to 10 wt%, or up to 5 wt% water-immiscible liquid compared to the total weight of the particle). One or more pharmaceutically acceptable carriers or excipients may also be provided in the core.

The particles of this embodiment, having no, or a small amount of water-immiscible liquid, have been found to be particularly responsive to ultrasound. Thus, they effectively generate inertial cavitation when exposed to ultrasound.

The core of the particle contains one or more therapeutic or diagnostic agents. The nature of the therapeutic or diagnostic agent which is present in the core is not particularly limited, and any agent which can usefully be delivered by means of the particles of the invention may be used. Where the core is a water-immiscible core as described herein, typically, the therapeutic or diagnostic agent is soluble in, or miscible with, the non-volatile components which make up the water-immiscible core. Thus, typically, the therapeutic or diagnostic agent is soluble in organic solvents. Certain therapeutic or diagnostic agents that are poorly soluble in organic solvents may be adapted to improve their solubility in organic medium. For instance, oligonucleotides such as DNA or RNA may be modified by techniques known in the art to render them soluble in organic media (e.g. as set out by Abe et al in Angewandte Chemie International Edition, volume 51, issue 26, 2012 page 6475-6479). Thus, the therapeutic or diagnostic agent referred to herein includes such derivatives.

Examples of therapeutic agents which may be contained in the core of the particles of the invention include analgesics, antibiotics, anti-thrombotic drugs (such as t-PA), antidepressants, anticancer drugs such as chemotherapy drugs including combinations thereof (e.g. 5-fluorouracil, folinic acid (FA) or salts thereof, 6-mercaptopurine, cytarabine, gemcitabine, oxaliplatin, methotrexate, actinomycin-D, bleomycin, daunorubicin, doxorubicin, docetaxel, estramustine, paclitaxel, vinblastine, etoposide, irinotecan, teniposide, topotecan, prednisone, methylprednisolone, dexamethasone and combinations of two or more of these chemotherapy drugs), antiepileptics, anti-inflammatory drugs, antipsychotic agents, antivirals, sedatives, steroids, antidiabetics, cardiovascular drugs, and drugs for pain management, treatment of skin conditions and treatment of brain diseases. In one embodiment, the therapeutic agent is imiquimod.

Examples of diagnostic agents which may be contained in the core include contrast agents and radioactive or tracer molecules. Particular examples of useful contrast agents are MRI contrast agents such as a Gd-based MRI contrast agents, or fluoroscopy contrast agents.

The particle of the invention may comprise a mixture of two or more different therapeutic or diagnostic agents. Typically, each particle contains a single therapeutic or diagnostic agent.

In one embodiment, the core does not contain an adjuvant. The term "adjuvant" as used herein refers to a substance which is used to enhance immunomodulation. Examples of adjuvants, which in this embodiment are typically not present in the core, include adjuvants soluble in an oil-based solvent. Thus in one embodiment, the core does not contain one or more of: aluminum salts such as alum, aluminum hydroxide or aluminum phosphate, calcium salts (e.g. calcium phosphate hydroxide), iron salts, zinc salts, an insoluble suspension of acylated tyrosine, acylated sugars, cationically or anionically derivatised saccharides, polyphosphazenes, biodegradable microspheres, monophosphoryl lipid A (MPL), lipopolysaccharides, lipid A derivatives (e.g. of reduced toxicity), 3-O-deacylated MPL [3D-MPL], detergents, e.g. quil A, Saponin, QS21, Freund's Complete Adjuvant, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, oils, e.g. squalene, mineral oils e.g. paraffin oil, food-based oils e.g. adjuvant 65 (derived from peanut oil), bacterial products, e.g. killed bacteria selected from *Bordatella pertussis, Mycobacterium bovis,* toxoids, bioadhesives and mucoadhesives, microparticles, liposomes, polyoxyethylene ether formulations, polyoxyethylene ester formulations, muramyl peptides or imidazoquinolone compounds (e.g. imiquamod and its homologues), human immunomodulators suitable for use as adjuvants including cytokines such as interleukins (e.g. 1L-1, IL-2, IL-4, IL-5, IL-6, 1L-7, IL-12, etc), macrophage colony stimulating factor (M-CSF), tumour necrosis factor (TNF), granulocyte and macrophage colony stimulating factor (GM-CSF). In a particular embodiment, the core does not contain an imidazoquinolone compound, e.g. imiquimod. In one embodiment, the core does not contain imiquimod or squalene or a mixture thereof.

The particle has a polypeptide shell, which typically surrounds the core, e.g. the polypeptide shell may surround a water-immiscible core or the polypeptide shell may surround the therapeutic or diagnostic component which is present in the core. The nature of the polypeptide is not particularly limited and a wide range of different polypeptides can be used.

Typically, the polypeptide shell comprises a polypeptide having two or more cysteine residues. This enables the polypeptide to form disulphide cross-linking bonds in order to generate the polypeptide shell. Preferably, the polypeptide contains at least 4 cysteine residues, for example at least 10 cysteine residues. A greater number of cysteine residues, in particular cysteine residues which are accessible on the surface of the polypeptide, generates a greater degree of cross-linking in the polypeptide shell, which may in turn provide greater stability to the structure. Cysteine residues are naturally occurring in many proteins. However, cysteine residues may be introduced into the polypeptide chain, for example by genetic engineering techniques.

The polypeptide shell typically comprises a polypeptide having a molecular weight of at least 5kD, preferably at least 10kD. A wide range of polypeptides having varying molecular weights can be used to form the polypeptide shell. Typically, therefore, the molecular weight of the polypeptide is from 5 to 80kD, preferably from 10 to 50kD.

Typically, the polypeptide shell comprises a protein. The polypeptide shell may comprise a human blood protein (including fragments thereof). Examples of suitable proteins include albumin, insulin, globulin and haemoglobin. Of these, albumin, insulin and globulin are preferred. In one embodiment, the polypeptide shell comprises a serum albumin protein, preferably human serum albumin. Human serum albumin, and other blood proteins, are non-immunomodulatory, i.e. they do not give rise to an immune response on administration to a human. They are therefore beneficial for use in cavitation agents where an immunomodulatory response is not desired.

In one embodiment, the polypeptide shell comprises one or more non-immunomodulatory polypeptides, typically one or more non-immunomodulatory proteins, i.e. one or more polypeptides or proteins which do not give rise to an immune response on administration to a human. In this embodiment, preferably the polypeptide shell contains no more than 5% by weight preferably no more than 1% by weight immunomodulatory protein, more preferably the polypeptide shell does not comprise an immunomodulatory protein. In this embodiment, preferably the polypeptide shell consists essentially of, preferably it consists of, one or more non-immunomodulatory polypeptides. A polypeptide shell which consists essentially of one or more non-immunomodulatory polypeptides comprises at least 95% by weight, preferably at least 98%, e.g. at least 99%, at least 99.5 or at least 99.9% by weight non-immunomodulatory polypeptide, or fragments thereof.

Preferably, in this embodiment, the polypeptide shell comprises a human blood protein, in particular serum albumin, insulin, globulin and/or haemoglobin. The shall may preferably comprise at least 95% by weight, preferably at least 98%, e.g. at least 99%, at least 99.5 or at least 99.9% by weight of one or more human blood proteins, preferably serum albumin, insulin, globulin and/or haemoglobin. Typically, no more than trace amounts of other proteins are present in the shell, such that the shell consists essentially of, in particular consists of, one or more human blood proteins, in particular it may consist essentially of, in particular consists of, one or more of serum albumin, insulin, globulin and/or haemoglobin.

Preferably, in this embodiment, the polypeptide shell comprises human serum albumin. More preferably, the polypeptide shell is a human serum albumin shell. A human serum albumin shell typically comprises at least 95% by weight, preferably at least 98%, e.g. at least 99%, at least 99.5 or at least 99.9% by weight human serum albumin, or fragments thereof. Typically, no more than trace amounts of other proteins are present in the shell such that the shell consists essentially of, in particular consists of, human serum albumin.

In an alternative embodiment, the polypeptide is immunomodulatory. The immunomodulatory polypeptide may be, for example, an immune checkpoint modulator, an antibody, a vaccine antigen, an adjuvant or an anti-inflammatory polypeptide. Further examples of immunomodulatory polypeptides useful in the present invention include macrophage colony stimulating factor (M-CSF), tumour necrosis factor (TNF, e.g. TNF-alpha), granulocyte macrophage colony stimulating factor (GM-CSF) and interferons.

Examples of suitable immune checkpoint modulators include anti-PDL-1 (aPDL1), anti-PDl (aPD1), anti-CTLA4 (aCTLA4), bi specific T-cell engagers (BiTE), cytokines and chemokines such as interleukins (e.g. IL-1, IL-2, IL-4, IL-5, IL-6, IL-7, IL-10, IL-12, etc.).

Examples of suitable antibodies include aPDL1; aPD1; aCTLA4; BiTE antibodies; anti growth factor antibodies that also trigger antibody dependent cellular cytotoxicity (ADCC) such as anti-EGFR antibodies (e.g. cetuximab and Herceptin); antibodies having anti-inflammatory effect such as anti-TNF (adalimumab, Humira); and JAK-inhibitor antibodies.

Examples of suitable antigens include vaccine antigens. For instance, the polypeptide shell may comprise at least one pathogenic antigen protein. The pathogenic antigen protein may be a full length protein or a fragment thereof. Examples of suitable antigen proteins include fragments of viral proteins or of parasitic proteins. The antigen may from a pathogen selected from Cal09 (flu), a coronavirus (e.g. Beta-coronaviridae or SARS-CoV-2), hepatitis B, *Bordatella pertussis* (whooping cough), *Streptococcus pneumoniae,* a meningococcus bacterium, human papilloma virus (HPV), or *Plasmodium* sporozoites (malaria parasite).

In one preferred aspect, the antigen protein is a viral spike protein, preferably a spike protein of the SARS-CoV-2 protein (e.g. SARS-CoV-2, S1 subunit protein (RBD)). In another aspect, the antigen is a circumsporozoite protein (CSP), a secreted surface protein of the sporozoite parasite. In a further aspect, the antigen is HepB surface antigen protein (HBsAg). In a further aspect, the antigen is associated with the influenza virus and is selected from haemagglutinin and/or an influenza neuraminidase. In another aspect, the antigen is filamentous haemagglutinin (pertussis). In another aspect, the antigen is pneumococcal surface protein A (PspA). In another aspect, the antigen is selected from Neisserial adhesin A (NadA), Neisserial Heparin Binding Antigen (NHBA) and/or factor H binding protein (fHbp). In another aspect, the antigen is an HPV-16 E6/E7 fusion protein. Fragments or genetically modified versions of any of these proteins may also be used.

Examples of suitable adjuvants include albumin proteins (e.g. bovine serum albumin, mouse serum albumin, ovalbumin), sFLT ligand, cytokines and chemokines such as interleukins (e.g. IL-1, IL-2, IL-4, IL-5, IL-6, IL-7, IL-12, etc), macrophage colony stimulating factor (M-CSF), tumour necrosis factor (TNF), and granulocyte and macrophage colony stimulating factor (GM-CSF).

Examples of suitable anti-inflammatory agents include adalimumab (Humira).

In one aspect, the polypeptide shell comprises an iron transfer protein (e.g. transferrin).

In one aspect, the particles do not contain an antigen, e.g. they do not contain a pathogenic antigen protein.

The polypeptide shell may comprise a single polypeptide, or a mixture of two or more different polypeptides. Preferably, the polypeptide shell comprises a single polypeptide (i.e. the polypeptide shell contains a single type of polypeptide, including fragments thereof, and does not contain a combination of two or more different polypeptides).

The polypeptide shell is optionally conjugated to one or more ligands. For example, the polypeptide shell may be conjugated to one or more ligands selected from a peptide, a protein, a sugar, a nanoparticle and a nucleic acid. Labelling moieties may also be conjugated to the polypeptide shell. Conjugation to proteins is commonly used for a variety of reasons. For example, the properties of the protein may be modified by conjugation to glycans (sugars) or to further proteins (e.g. antigens) having specific desired properties. Labelling moieties may be provided to enable imaging of the particles, for example as part of a diagnostic assay.

A common technique for conjugation to proteins, which can be employed in the present invention, is to target lysine residues on the protein surface. Thus, ligands having a group reactive with amine (e.g. NHS) may be conjugated to lysine on the polypeptide shell. However, alternative conjugation chemistries may be employed.

The ligands may be conjugated to the polypeptide shell after particle formation. Alternatively, the ligand may be conjugated to the polypeptide before formation of the particle.

The shell thickness of the polypeptide particle is typically at least 10nm, preferably at least 20nm, for example about 30-40nm. For example, the polypeptide shell thickness may be from 10 to 100nm, preferably from 20 to 60nm. The thickness of the polypeptide nanoparticle shell can be determined by cryo-slicing and TEM imaging.

The particles of the invention are highly stable and can be stored as a suspension in aqueous solution or in solid form. For instance, the particles can be stored for at least a period of several months at 4°C.

### Synthesis

The polypeptide particles of the invention can be made by a two step process which involves (a) obtaining particles having a polypeptide shell and a water-immiscible core comprising one or more volatile components and optionally one or more non-volatile components, and (b) removing the volatile component from the core in order to create the desired indented particle having ultrasound-responsive properties.

The invention therefore provides a method of producing a polypeptide particle according to the invention, comprising:
- providing a water-immiscible phase comprising one or more volatile components, one or more therapeutic or diagnostic agents and optionally one or more non-volatile components;
- mixing said water-immiscible phase with an aqueous solution of at least one polypeptide comprising at least two cysteine residues, thus providing a biphasic composition;
- cross-linking the cysteine residues to generate a particle having a core comprising the water-immiscible phase and a shell comprising the at least one polypeptide; and
- creating one or more indentations in the particle by subjecting the particle to reduced pressure conditions, to remove volatile component from the core.

The particles are typically produced by first creating a biphasic composition comprising (1) a water-immiscible phase comprising one or more volatile components together with one or more therapeutic or diagnostic agents and optionally one or more non-volatile components; and (2) an aqueous phase comprising the polypeptide or mixture of polypeptides which is to be present in the polypeptide shell. The aqueous solution typically comprises at least about 0.05% w/v of polypeptide, for example at least about 0.5%, preferably at least about 1% w/v polypeptide. Typically the polypeptide concentration is no higher than about 25% w/v, e.g. no higher than about 15% w/v, preferably no higher than about 10 %w/v. Thus, suitable concentrations of polypeptide in the aqueous phase are from 0.5 to 15% w/v, preferably from 1 to 10% w/v, more preferably about 5% w/v.

The volumetric ratio of water-immiscible phase to aqueous phase in the biphasic mixture is typically about 1:5 to 3:1, for example from 1:4 to 1:1, e.g. about 1:3.

The water-immiscible phase comprises one or more volatile components. The nature of the volatile component(s) is not particularly limited, but is typically selected so that the or each volatile component is (a) water-immiscible, typically it is a volatile organic component, e.g. a volatile organic solvent, and (b) easily removed in the reduced pressure step. Typically, therefore, the or each volatile component has a vapour pressure at 25°C which is greater than that of water at the same temperature. Typically, the or each volatile component has a vapour pressure at 25°C of at least 3 kPa, preferably at least 3.5 kPa, for example at least 5 kPa, at least 10 kPa or at least 15 kPa. at 25°C. Suitable volatile components include hexane, chloroform, ethyl acetate, propyl acetate and toluene. Mixtures of two or more of these components can be used.

The volatile component may initially be used as a solvent for the therapeutic or diagnostic agent(s), such that a solution of therapeutic or diagnostic agent in the volatile component(s) is used to produce the particles of the invention. Any pharmaceutically acceptable excipients which are used may also be dissolved in the volatile component(s).

The biphasic mixture may be held at elevated temperature for a period of time prior to the next step. For instance, the biphasic mixture may be incubated at around 37°C for up to 2 hours, for example for about 1 hour.

The biphasic mixture comprising the water-immiscible phase and aqueous phase is then subject to conditions which cause the cysteine residues in the polypeptide to cross-link. The conditions used may also emulsify the mixture. Typically, for example, cross-linking is achieved by homogenising the bi-phasic mixture e.g. by applying high pressure and/or high shear stress conditions. Preferably, homogenisation is carried out by use of ultrasound. The high shear stress causes cross linking of the polypeptide, in particular by connecting cysteine groups within the polypeptide chains. It is understood that the homogenisation conditions may oxidise the sulfhydryl groups on a cysteine residue, or disrupt existing disulphide bonds, thereby allowing new disulphide bonds to form and generating the cross-linked shell structure. Water-immiscible phase remains physically trapped within the cross-linked polypeptide shell, providing the desired core-shell structure. The particles produced by the homogenisation step are generally substantially spherical in shape.

One example of a method for achieving homogenisation is to place an ultrasound horn at the interface of the aqueous and water-immiscible phases, and apply low-frequency ultrasound. Ultrasound may be applied at around 50% amplitude. For example, ultrasound may be applied for at least 1 minute, e.g. about 3 minutes. For example, particle formation has been achieved using a QSonica Q125 ultrasound probe (frequency: 20kHz; power: 125 watts) at 50% amplitude for 3 minutes.

An alternative method for achieving homogenisation is to use hydrodynamic cavitation, which uses high pressure to pump a liquid through a narrow orifice. The increase in flow velocity as the liquid passes through the orifice, and subsequent decrease in velocity once past the orifice, causes cavitation nuclei to grow and then collapse. Applying hydrodynamic cavitation to the biphasic mixture may cause cross-linking of cysteine residues and/or emulsification, leading to formation of particles. Hydrodynamic shearing is a further alternative method which may produce the same reactive oxygen species that reduce / oxidise cysteine residues that lead to particle formation. Other methods of sonication may also be used.

Spherical particles having a water-immiscible core and a polypeptide shell, which are produced by such homogenisation methods, are known in the art, and methods for their synthesis have been previously described (see Suslick K. S. and M. W. Grimstaff, Journal of the Americal Chemical Society, 1990, 112(21), p7807-7809, the contents of which are incorporated herein by reference). The particles of the invention may be made by such techniques or other methods of sonication, such as those described in US5439686, the contents of which are incorporated herein by reference.

Following the creation of the polypeptide shell, the particles of the invention undergo a step in which the volatile component(s) are removed, causing deformation of the particle and thus the desired indented structure. Removal of the volatile component(s) can be done under reduced pressure conditions. For instance this step can be carried out using a vacuum protein concentrator such as a Speed Vac^{™} (Thermo Scientific), but other instrumentation such as a rotary evaporator can be used. Reduced pressure conditions are typically applied for at least 30 minutes, preferably at least an hour. For instance, reduced pressure may be applied for from 2 to 16 hours.

The step of removing volatile component removes all or part of the volatile component(s) present. Preferably at least 50%, more preferably at least 80%, 90% or 95% by volume of the volatile component are removed. More preferably substantially all (e.g. at least 98%, 99% or 99.5% by volume) of the volatile components are removed. Removal is sufficient to create an indented structure.

The nature and amount of volatile component which is present in the water-immiscible phase affects the particle size and may be used as a factor in controlling the size of particle produced. The amount of volatile component also affects the deformation of the particle and thus the size of any indentation. In general, the greater the amount of volatile component, the greater the size of the indentation which is expected to be produced. Variation in the size of the indentation may also be relevant to the cavitation effects generated by the indented particle, due to the change in the potential for gas bubbles to be trapped within the cavity of the indentation.

Where both volatile and non-volatile components are used in the water-immiscible phase, the amount of volatile component can be varied by changing the relative amount of non-volatile and volatile component in the water-immiscible phase. Typically, the volume ratio of (a) volatile component containing dissolved therapeutic or diagnostic component, to (b) non-volatile component is from about 1:20 to 20:1, preferably from about 1:4 to 15:1, e.g. about 1:3 to 3:1, more preferably about 0.5:1 to 2:1 (1:2 to 2:1), most preferably about 1:1. A minimum volume ratio of at least 1:20 of volatile to non-volatile component (about 5% by volume non-volatile component) is typically needed to achieve indentation in the particle on removal of the volatile component form the core. A volatile content of at least 40% by volume, preferably from 45 to 55% by volume in the water-immiscible phase, preferably around 50% by volume (or 1:1), has been found to provide improved cavitation properties.

Alternatively, the water-immiscible phase may comprise substantially no non-volatile component, or non-volatile component may be absent. Thus, there may be no more than 5% by volume, for example no more than 2%, 1% or 0.5% by volume non-volatile component, for example no more 0.1% or 0.01% by volume non-volatile component.

The amount of the therapeutic or diagnostic agent which is present in the water-immiscible phase may vary and depends on the nature of the agent. For instance, the therapeutic or diagnostic agent may be present in an amount of from 0.1 ug to 1 mg/ml, typically from 0.5 to 100ug/ml, e.g. from 1 to 10ug/ml in the volatile component. The amount of therapeutic or diagnostic component which is present in the core of the particle once produced can be controlled by varying the concentration of the agent in the volatile component.

The method of the invention may further comprise the steps of:
- drying the particle; and
- optionally re-suspending the particle in a liquid medium.

The drying step may be carried out by evaporation, e.g. under reduced pressure and/or with mild heating. For instance this step can be carried out using a rotary evaporator set to a temperature of up to about 40C. Typically, the drying step is combined with the step of evaporation of volatile component, but continuing the reduced pressure (and optionally mild heat) conditions for a longer period of time and by use of mild heating, in order to evaporate water to provide a solid product. Thus, for instance, removal of volatile component and drying may be carried out by applying reduced pressure conditions and heating at up to 40C until a solid is produced.

The particles are then preferably re-suspended in a liquid medium, typically water or an aqueous solution such as a buffer. Re-suspension may provide improved long term stability to the particles and they are therefore preferably re-suspended prior to storage. However, particles may alternatively be stored in solid form and re-suspended prior to use. Drying and re-suspension may assist in trapping gas bubbles in any indentations on the particle, which provides improved inertial cavitation response.

The method may also comprise:
- lyophilising the particle; and
- optionally re-suspending the particle in a liquid medium.

Typically, the particles are re-suspended in a liquid medium, typically water or an aqueous solution such as a buffer. Re-suspension may provide improved long term stability to the particles and they are therefore preferably re-suspended prior to storage. However, particles may alternatively be stored in solid (lyophilised) form and re-suspended prior to use. Lyophilisation and re-suspension may further assist in trapping gas bubbles in any indentations on the particle, which provides improved inertial cavitation response. A particularly improved cavitation response has been demonstrated with particles which have been both dried and re-suspended, and subsequently lyophilised and re-suspended.

The additional steps of drying and lyophilisation may be carried out with any of the particles described herein. However, in a preferred embodiment, where the particle is produced by use of only volatile component(s) in the core (i.e. the final particle after evaporation of volatile component does not comprise a water-immiscible liquid in the core), or where the particle is produced using a low proportion, typically less than 10% by volume, preferably less than 5% by volume, e.g. less than 1% or less than 0.1% by volume, of non-volatile component in the water-immiscible phase (such that the final particle has a core comprising a small amount of non-volatile component, e.g. less than 10 wt%, preferably less than 5 wt% compared to the total particle), it is preferred that the particles are dried and/or lyophilised. Most preferably, these particles are dried, re-suspended and then lyophilised. Yet more preferably, these particles, are dried, re-suspended, lyophilised and again re-suspended.

The particles can be purified by suitable means and/or subject to size filtration. For example, particles can be separated from residual oil, solvent and protein by any suitable means, for example, by dialysis e.g. through a dialysis filter having a molecular weight cut off of appropriate size (e.g. 1M Da). Size filtration and/or centrifugation may also be used to limit the maximum size of the particles. Purification may be carried out following evaporation of the volatile component, following drying and re-suspension, or following lyophilisation and re-suspension.

The particles may be suspended in a liquid medium for storage or use, e.g. water, or an aqueous solution, optionally a buffer solution.

### Compositions

The present invention provides a composition, typically a pharmaceutical composition, comprising one or more polypeptide particles according to the invention. The composition may comprise a plurality of the polypeptide particles alone, or the particles may be provided together with one or more pharmaceutically acceptable carriers or diluents. Typically, compositions provided together with a carrier or diluent contain up to 85 wt% of a particle of the invention. More typically, such a composition contains up to 50 wt% of a particle of the invention. Preferred pharmaceutical compositions are sterile and pyrogen free.

The compositions of the invention may comprise one or more different types of particle according to the invention. For instance, the composition may comprise first particles having a first type of polypeptide shell and second particles having a second (different) type of polypeptide shell. Typically, the compositions of the invention comprise a single type of particle. Similarly, the composition may comprise first particles having a first (combination of) therapeutic or diagnostic agent in the core and second particles having a second (combination of) therapeutic or diagnostic agent in the core.

The particles in the composition may be substantially monodisperse. For instance, they may have a polydispersity index of 0.20 or less, e.g. 0.19 or less, 0.18 or less, 0.17 or less, 0.16 or less, or 0.15 or less. Greater monodispersity of the particles may provide a greater control over cavitation and thus improved acoustic tuning. Polydispersity may be determined by DLS.

The particles of the invention may be administered by any suitable route, depending on the nature of the treatment, e.g. orally (as syrups, tablets, capsules, lozenges, controlled-release preparations, fast-dissolving preparations, etc); topically (as gels, creams, ointments, lotions, nasal sprays or aerosols, etc); by injection (subcutaneous, intradermal, intramuscular, intravenous, intratumoural, intrathecal etc.), transdermally (e.g. by application of a patch, gel or implant) or by inhalation (as a dry powder, a solution, a dispersion, etc). In one aspect, the particles are administered parenterally, e.g. by infusion or by injection, preferably by intravenous, intramuscular, intradermal or subcutaneous administration. In another aspect, the particles are administered by transdermal administration. Preferably, the particles are administered parenterally, e.g. by injection (preferably intravascularly, or intrathecally), or transdermally.

For example, solid oral forms may contain, together with the particles of the invention, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

In one embodiment, a composition provided together with a carrier or diluent is a liquid dispersion. Typically, water (preferably sterile water) is used as diluent for a liquid dispersion. Liquid dispersions for oral administration may be syrups, emulsions and suspensions. Suspensions are preferred. The syrups, emulsions and suspensions may contain as carriers, for example, saccharose or saccharose with glycerine and/or mannitol and/or sorbitol. Glucose is a preferred carrier. For example, the particles may be provided as a suspension in a glucose solution, for example a glucose solution comprising up to 10wt% glucose, e.g. about 5wt% glucose.

Suspensions and emulsions may contain as carrier, for example a natural gum, agar, sodium alginate, pectin, methylcellulose, carboxymethylcellulose, or polyvinyl alcohol. The suspensions or solutions for parenteral administration, e.g. intramuscular injection, may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Particles for administration in suspension form may be provided as lyophilised particles, for reconstitution in a suitable carrier (e.g. sterile water) prior to administration.

A gel for transdermal administration may comprise a diluent selected from an alcohol such as ethanol or propanol, glycerine, propylene glycol or polyethylene glycol. The gel may also comprise preservatives and may comprise water or a buffer solution. Permeation enhancers such as fatty acids, fatty alcohols, urea, sulphoxides, azone, pyrrolidones, essential oils, terpenes and terpenoids may be incorporated. Alternatively the gel for transdermal delivery may be formulated as a proniosome or microemulsion gel.

### Administration

As discussed above, the particles of the invention can be administered via a variety of routes of administration. Once administered, ultrasound is provided at the target site in order to generate the inertial cavitation response of the particles.

Ultrasound is typically delivered at a frequency of from 100 to 3000kHz, for example from 100 to 1000kHz, for example from 200 to 600kHz and optionally a duty cycle (DC) of from 1% to 20%, e.g. from 2% to 15%. Typically, the ultrasound delivered has a pulse repetition frequency (PRF) of from 0.01 to 100 Hz, e.g. from 0.1 to 50Hz, preferably from 0.1 to 20Hz. Typically, the ultrasound is provided at a peak negative pressure (PNP) of 0.5 MPa to 7.0 MPa, e.g. from 0.5 to 3.0 MPa, e.g. from 1.0 to 2.5 MPa, for example at least 1.5MPa or from 1.5 to 2.5MPa.

Particularly preferred ultrasound conditions are from 1.0 to 2.0MPa and 100 to 600kHz.

The ultrasound parameters may be varied depending on the mode of administration. For instance, for transdermal delivery, the ultrasound may be delivered at a frequency of from 100 to 500kHz, for example from 200 to 300kHz and optionally a duty cycle (DC) of from 5% to 20%, e.g. from 5% to 15%. Typically, the ultrasound delivered has a pulse repetition frequency (PRF) of from 0.1 to 100 Hz, e.g. from 1 to 50Hz, preferably from 5 to 20Hz. Typically, the ultrasound is provided at a peak negative pressure (PNP) of 0.5 MPa to 2.0 MPa, e.g. from 1.0 to 2.0 MPa, 1.5MPa to 2.0MPa.

For parenterally administered, e.g. injected particles, ultrasound is typically delivered at a frequency of from 200 to 3000kHz, for example from 200 to 1000kHz, for example from 400 to 600kHz and optionally a duty cycle (DC) of from 1% to 10%, e.g. from 2% to 10%. Typically, the ultrasound delivered has a pulse repetition frequency (PRF) of from 0.01 to 50 Hz, e.g. from 0.1 to 10Hz, preferably from 0.1 to 1Hz. Typically, the ultrasound is provided at a peak negative pressure (PNP) of 1.0 MPa to 7.0 MPa, e.g. from 1.0 to 3.0 MPa e.g. from 1.5 to 2.5 MPa.

Ultrasound may be applied for a duration of from 10 seconds to 15 minutes, typically from 30 seconds to 10 minutes, preferably from 1 to 5 minutes.

Exposure of the particles to ultrasound after administration causes an inertial cavitation effect in the fluid in which the particles are contained. For instance, if the particles are administered systemically, e.g. intravascularly or intra-arterially, exposure of the particles to ultrasound at a target site causes inertial cavitation within the vasculature. This may lead to a miscrostreaming effect in which the particles themselves, and any contained therapeutic or diagnostic agents, may be transported to a target site. In this way, inertial cavitation can be used to selectively target chosen sites of administration.

Similarly, application of ultrasound to a formulation of particles applied to the skin may cause inertial cavitation within the formulation, assisting in the transdermal delivery of the particles, and any contained diagnostic or therapeutic agent. Ultrasound has previously been used to assist transdermal delivery of drugs. However, previous work has focussed on the use of ultrasound to permeabilise the skin and particles for delivery have been administered with additional means, for example via ballistic delivery. In contrast, the present invention enables particles to travel through the skin solely by applying the particles in a suitable formulation, such as a gel, and providing ultrasound at the site. The combination of the small size of the particles, and the microstreaming effect caused by inertial cavitation, pushes the particles through the relatively impermeable stratum corneum, thus providing effective transdermal delivery.

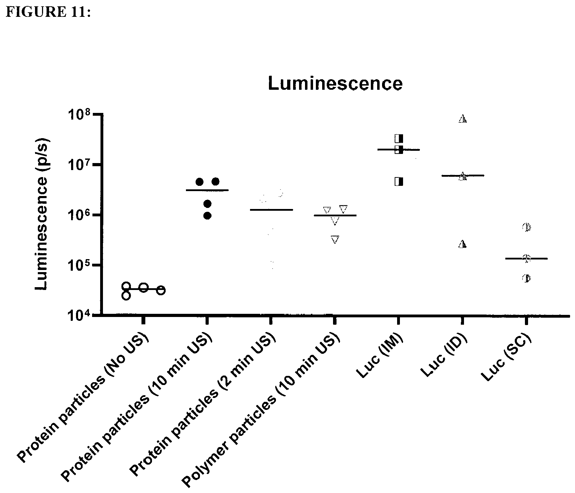

The examples provided herein demonstrate the successful administration of the particles of the invention via transdermal delivery. Particles were administered together with DNA encoding luciferase and post-administration luminescence measurements provided an indication of the degree of successful transdermal administration (see Figure 11). A greater degree of luminescence was observed following administration with cavitation agents described herein than with prior art cavitation agents. Luminescence was comparable to that achieved by direct injection of DNA.

### Use of Particles

The particles of the invention are useful in the cavitation assisted delivery of therapeutic and diagnostic agents to target sites in the body. The use of inertial cavitation to accumulate nanoparticles in selected sites has previously been demonstrated by Kwan et al in Small, 2015 Oct; 11(39): 5305-5314. The particles of the present invention, having the ability to generate inertial cavitation effects in vivo, similarly can be accumulated at a selected target site, such as a tumour. The invention thus provides an accumulation of therapeutic or diagnostic agent in a desired target site.

Application of ultrasound following administration of the nanoparticle causes cavitation to occur. This enhances transport of the contained therapeutic or diagnostic agent into cells. A particular advantage of using cavitation to enhance transport of an active agent into cells in this way is that ultrasound can be applied at selected locations, leading to the active agent being transported into cells only in those selected locations. Furthermore, the use of inertial cavitation to enhance transport may increase the amount of agent which reaches the target site. Biological degradation of the protein may release the contained therapeutic or diagnostic agent selectively at the target site. The particles of the invention are therefore useful in increasing delivery of an agent to a target site.

The invention therefore provides particles for use in enhancing transport of the therapeutic or diagnostic agent to a target site. The invention also provides a method for enhancing transport of the therapeutic or diagnostic agent to a target site. Also provided is the use of a particle or composition as described herein in the manufacture of a medicament for use in enhancing transport of the therapeutic or diagnostic agent to a target site. Such enhanced transport increases the extracellular or intracellular concentration of the therapeutic or diagnostic agent. The enhanced transport is achieved via the microstreaming effect of inertial cavitation. Particles are physically driven through natural holes between cells, for example natural holes in the endothelium. This therefore provides an improved method of transporting therapeutic or diagnostic agent compared with, for example, methods which increase the permeability of the endothelium.

Where the particles of the invention are to be used to deliver agents to an extravascular location, e.g. to a tumour, the particles preferably have a size of up to 500nm, typically from 100nm to 500nm. More preferably the particles have a size of from 100 to 300nm. Such particle sizes may, for example, be desired in order to improve accumulation in tumour tissue by the enhanced permeability and retention (EPR) effect. Tumour tissues may contain neovasculature having abnormal form and architecture, leading to abnormal molecular and fluid transport dynamics. That can cause nanoparticles of around 100 to 500 nm, e.g. 100 to 300 nm in size to accumulate in tumour tissue much more than they do in normal tissues. Nanoparticle sizes of 100 to 500 nm, e.g. 100 to 450nm or 100 to 300 nm may therefore be preferred, in particular for use in treating tumour. In a preferred embodiment, where the nanoparticles are for use in treating tumour, the particle size is 450nm or less.

The particles may be administered transdermally and are therefore also useful in the treatment of diseases or disorders of the skin. They are also useful in the treatment of skin injury or infection (e.g. where the particles contain one or more anitbiotics, antivirals and/or analgesics).

The particles are particularly useful in the treatment of tumour (e.g. where the particles contain one or more chemotherapeutic agents such as those discussed herein). In this instance, ultrasound may be used to target drug to the tumour. The particles are also useful in the treatment of cardiovascular disease, particularly thrombolytic diseases, e.g. ischaemic stroke or deep vein thrombosis (e.g. where the particles contain one or more antithrombotic agents such as tPA). In this case, ultrasound may be used to target drug for delivery to the heart. The particles are also useful in the treatment of infection (typically bacterial infection), for example urinary tract infection and infections which can occur following bone fracture (e.g. where the particles contain one or more antibiotics). In this case, ultrasound may be used to target the site of infection or injury. The particles may also be useful in the treatment of neurological diseases and disorders such as Alzheimer's disease, Parkinson's disease and Essential Tremor as well as other neurological diseases (e.g. where the particles contain one or more antidepressants, antiepileptics, antipshychotic agents, or drugs known to be associated with cognitive improvement). In this case, ultrasound may be used to target delivery of drugs to the brain. The use of ultrasound assisted delivery may have the additional benefit of opening the blood brain barrier.

The particles can also be used in any situation in which inertial cavitation is desired such as histotripsy and thermal ablation, in particular where such methods are usefully accompanied by administration of therapeutic agents, or monitored by the administration of diagnostic agents such as contrast agents. For example, the particles can be for use in methods that involve using inertial cavitation to cause localised tissue ablation and/or removal in order to achieve a therapeutic outcome. Such methods include for example the treatment of tumours and removal of cardiac tissue in treatment of heart disease. In this aspect, the particles of the invention are advantageous due to their small size, stability, and biodegradeability.

The present invention therefore provides particles and compositions as described herein for use in a method of diagnosis or therapy. The method typically comprises administering a particle or composition as described herein and applying ultrasound. In particular, the particle or composition is for use in generating inertial cavitation in the subject. Inertial cavitation can be generated at a chosen target site such that the particles are useful in providing transport of therapeutic or diagnostic agent to the target site. Also provided is a method of treatment of a subject, the method comprising administering an effective amount of a particle or composition as described herein and applying ultrasound. The method may generate inertial cavitation in the subject. Also provided is the use of a particle or composition as described herein in the manufacture of a medicament for use in treatment by therapy or diagnosis, the method comprising administering the particle or composition and applying ultrasound. In particular, the medicament is for use in generating inertial cavitation in the subject.

The nanoparticles can also provide real time tracking and mapping of drug distribution *in vivo,* for example using conventional ultrasound imaging or passive acoustic mapping of inertial cavitation (WO 2010 052494).

### Dosages

The dose of particles of the invention may be determined according to various parameters, especially according to the substance used; the age, weight and condition of the individual to be treated; the route of administration; and the required regimen. A physician will be able to determine the required route of administration and dosage for any particular individual. The dose may be provided as a single dose or may be provided as multiple doses, for example taken at regular intervals, for example 2, 3 or 4 doses administered hourly. Typically polypeptide-based treatments are administered in the range of 1 pg to 1 mg, more typically 1 pg to 10 µg for particle mediated delivery and 1 µg to 1 mg, more typically 1-100 µg, more typically 5-50 µg for other routes. Generally, it is expected that each dose will comprise 0.01-3 mg of polypeptide.

### Examples

### Particle Synthesis

Ultrasound-responsive particles were prepared according to protocol A as follows:
3ml of a 50 mg/ml solution of protein is overlaid with 1ml of a water immiscible solution containing an oil, and optionally a volatile component and in some cases an additional agent, to produce a biphasic solution. The biphasic mixture is sealed in a glass vial and placed in an incubator at 37C for 1 hour.

An ultrasound horn is then placed at the interface of the two layers and low frequency ultrasound at 50% amplitude is applied for 3 minutes at 125 watts using QSonica Q125 at 50% amplitude. This produced an emulsion of particles comprising a core containing the water immiscible mixture and a protein shell.

The volatile solvent component of the particle core is then removed under reduced pressure using a SpeedVac^{™} protein concentrator to provide a solution containing the particles.

The particles are then separated from residual oil, solvent and protein by dialysis in a 1M Da molecular weight filter for 24 to 48 hours.

Particles were also produced according to a modified protocol B. Protocol B is identical to protocol A with the exception that the protein was at a lower concentration of 0.5mg protein in 666ul of water which was then overlaid with 333ul of the oil/volatile solvent layer.

Particles were also produced according to a modified protocol C:
3ml of a 50 mg/ml solution of protein is overlaid with 1ml of a water immiscible solution to produce a biphasic solution. The biphasic mixture is sealed in a glass vial and placed in an incubator at 37C for 1 hour.

An ultrasound horn is then placed at the interface of the two layers and low frequency ultrasound at 50% amplitude is applied for 3 minutes at 125 watts using QSonica Q125 at 50% amplitude. This produced an emulsion of particles comprising a core containing the water immiscible mixture and a protein shell.

The volatile solvent component of the particle core is then removed under reduced pressure by rotary evaporation at 40C for 48 hours to provide solid particles.

Particles are re-suspended in water. Some particles are then freeze-dried for 48 hours, followed by re-suspension in water once again.

The following particles were produced:

**Table 1**

| Ex. No | Protocol | Water-immiscible layer | | | | Protein |
|---|---|---|---|---|---|---|
| | | Oil | Volatile | % volatile | Agent added | |
| 1 | A | Sunflower oil | Hexane | 50 | None | Bovine Serum Albumin (BSA) |
| 1A | A | Sunflower oil | Hexane | 50 | 300ug Nile Red | BSA |
| 1B | A | Sunflower oil | Chloroform | 50 | 0.5mg Terazonsin | BSA |
| 2 | A | Sunflower oil | none | 0 | None | BSA |
| 3 | A | Sunflower oil | chloroform | 50 | None | BSA |
| 4 | A | Sunflower oil | Ethyl acetate | 50 | None | BSA |
| 5 | A | Sunflower oil | Propyl acetate | 50 | None | BSA |
| 6 | A | Sunflower oil | toluene | 50 | None | BSA |
| 7 | A | Sunflower oil | chloroform | 90 | None | BSA |
| 8 | A | Sunflower oil | Ethyl acetate | 90 | None | BSA |
| 9 | A | Sunflower oil | Propyl acetate | 90 | None | BSA |
| 10 | A | Sunflower oil | toluene | 90 | None | BSA |
| 11 | A | Sunflower oil | Hexane | 30 | None | BSA |
| 12 | A | Sunflower oil | Hexane | 70 | None | BSA |
| 13 | A | Sunflower oil | Hexane | 90 | None | BSA |
| 14 | A | Sunflower oil | Hexane | 50 | None | Ovalbumin (≥99% purity, Sigma A5503) |
| 15 | A | Sunflower oil | Hexane | 50 | None | IgG |
| 16 | A | Sunflower oil | Hexane | 50 | None | Transferrin (≥95% Sigma 90190) |
| 17 | B | Sunflower oil | Hexane | 50 | None | Mouse Serum Albumin (≥96% purity, Sigma A3139) |
| 18 | B | Sunflower oil | Hexane | 50 | None | Covid spike protein |
| 19 | B | Sunflower oil | Hexane | 50 | None | Cetuximab (also known as Erbitux), Merck, Pharmaceutical grade, 5mg/ml |
| 20 | C | None | Hexane | 100 | None | BSA |

Bovine serum albumin (BSA) used was ≥99% purity, Sigma A7638.

IgG was non-specific IgG isolated form human blood ((≥99% Sigma, 14506).

Covid spike protein was SARS-CoV-2, S1, RBD, Cambridge Bioscience, 230-30162-1000.

### Particle Analysis and Chemical Modification of Particle Surface

Particles were analysed by dynamic light scattering (DLS), scanning electron microscopy (SEM) and via spinning disc confocal microscopy following conjugation of the particle to fluorophores.

### SEM analysis

Instrumentation:
▪ Scanning electron microscopy (SEM) analysis was performed using a Zeiss Sigma 300 Field Emission Gun Scanning Electron Microscope (FEG-SEM).

Method:
▪ Typically a 1 in 1000 dilution of a stock solution was made. This is fixed with 1% glutaraldehyde for 2h, and 10ul added to a black carbon tape and coated with gold before imaging

### Spinning disc confocal microscopy

Instrumentation:
▪ An Andor Dragonfly spinning disc confocal microscope was used to generate high resolution Z stacks that were then compiled into 3D reconstructions of protein particles

Method:
▪ Particles were reacted with CY-5^{®} N-hydroxysuccinimide (NHS) or AlexaFluor^{™} 488 N-hydroxysuccinimide (NHS) for 1h. Dialysis for 16h using a 10kDa molecular weight cassette was used to remove residual unconjugated dye prior to imaging.

### Dynamic Light Scattering

Instrumentation:
▪ Size distributions were measured using DLS (ZetaSizerNano, Malvern) with a red laser. Each sample was tested with three measurements of approximately 10 runs.

Method:
▪ 10ul of a particle sample prepared as set out above was diluted to 1ml with Millipore water and analysed in a standard 1ml cuvette.

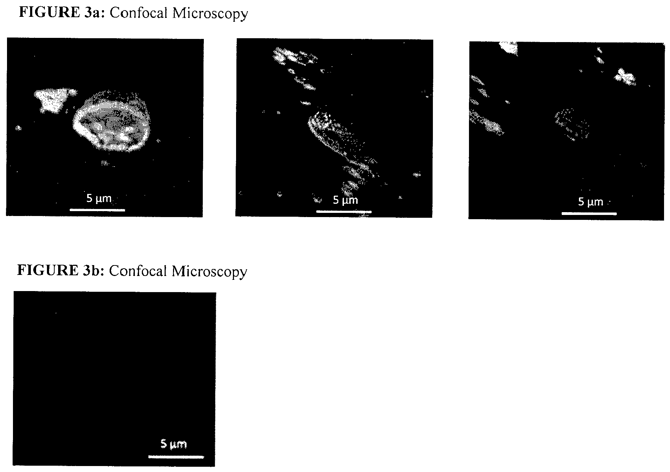

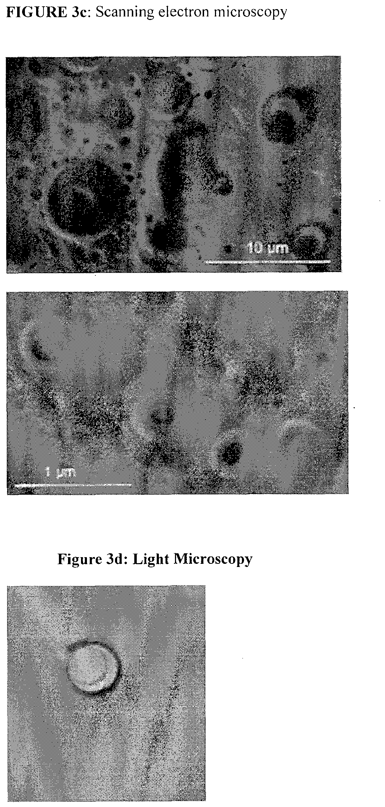

Figures 3a to 3b and Figure 3c show the particles of Example 1 above via 3D confocal microscopy and SEM respectively. Ligands successfully conjugated to particles via NHS-lysine conjugation, and provided visibility of particles via confocal microscopy as shown in Figure 3a.

The particles can be seen to have an indented structure, consistent with the ability of the particle to trap a gas bubble in the indentation. The particles have either one or two major indentations.

This experiment also confirms that upon forming particles, the protein retains the ability to undergo standard protein conjugation reactions, in this case reaction with chemistry targeting lysine residues. The particles became fluorescent following conjugation of two different lysine reacting dyes.

Figure 3d shows a particle according to Example 20 (following freeze-drying and resuspension in water) viewed using light microscopy.

### Addition of Compound to Oil Core

Example 1A demonstrates that compounds dissolved in the oil layer during synthesis are incorporated into the oil core of the particles.

Protocol A was followed to produce the particles as set out in Example 1 above, but 300ug of Nile Red oil soluble fluorophore was incorporated into the oil layer prior to application of ultrasound to form the particles. Any residual fluorophore was removed from the particles by dialysis.

Fluorescent confocal microscopy was used to image the particles. This confirmed the presence of Nile Red inside the core of the particles.

Example 1B describes the production of particles containing the therapeutic drug terazonsin. Terazonsin was included in the chloroform volatile component at a concentration of 1 mg/ml and mixed with the sunflower oil prior to particle production. Protein particles were produced according to the standard protocol A.

Purified protein particles were digested with beta mercaptoethanol and proteinase K for 1 hour at 60C to break up the external protein shell and release the inner core. The core content was then analysed by UPLC to confirm the presence of terazonsin. UPLC analysis was carried out substantially as described in the method by Balireddi et al (Hournal of Chrom Science, Volume 57, Issue 3, March 2019, p249-257).

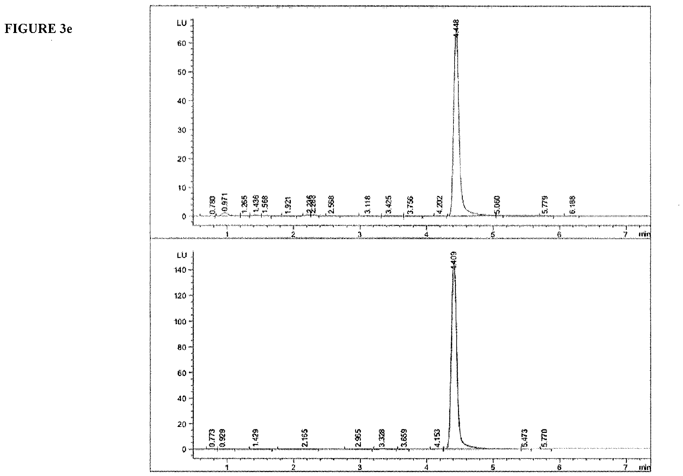

UPLC analysis confirmed the presence of terazonsin as shown in Figure 3e. The top panel of Figure 3e shows UPLC analysis of the digested protein particles, whilst the bottom panel shows a reference standard of terazonsin. Detection was via fluorescence with Ex=280 and Em=413nm.

### Effect of Volatile Component on Particle Size

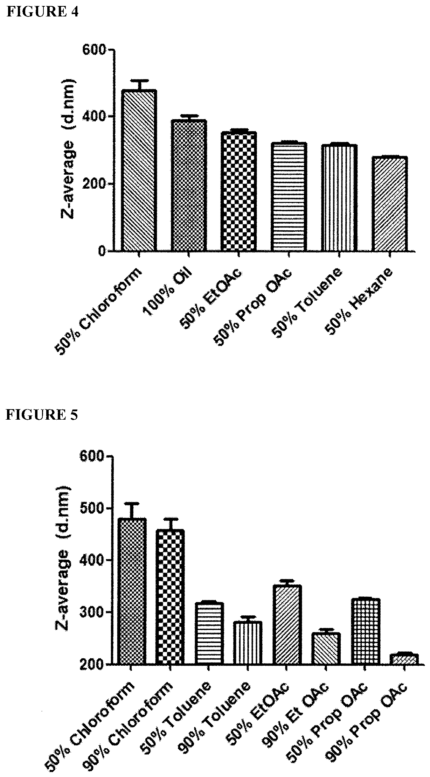

The nature of the volatile component was varied in Examples 3 to 6, in order to determine the effect on the particle size. Results are depicted in Figure 4, showing that variation of the volatile component can be used to vary the particle size. Similarly, the amount of the volatile component was varied in Examples 7 to 10, and the change in particle size studied. The results are depicted in Figure 5. Increase in the amount of volatile component generally leads to a reduction in particle size.

### Variation of Polypeptide

Variation of the protein to incorporate a variety of different proteins into the particle was achieved as set out in Examples 14 to 19.

### In Vitro Cavitation of Particles

All measurements of inertial cavitation were carried out at a frequency of 0.5 MHz, 1000 cycle pulse length, and 100 ms pulse repetition period, with varying driving pressures as set out below.

First, to characterise the cavitation ability of particles across a range of sizes, particles produced according to Example 1 above were filtered through 200nm and 450nm filters and then assessed for inertial cavitation. The passive cavitation detection (PCD) level was assessed at driving pressures of 0.5MPa, 1.0MPa, 1.5MPa and 2.0MPa. Time averaged PCD levels over a 30 second exposure are provided in Figure 6 for particles without size filtration (all sizes), and particles filtered through 200nm and 450nm filters. Cavitation was detected in all particles.

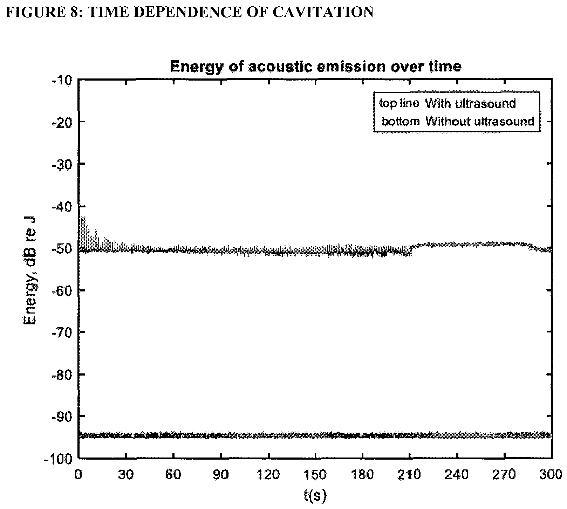

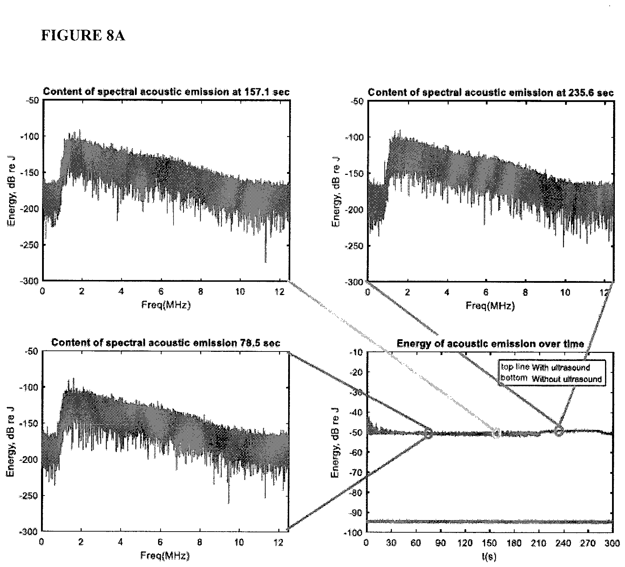

Inertial cavitation was also demonstrated, using particles produced according to Example 18 above. Particles were exposed to 1.2 MPa for 5 minutes. Figure 8 shows a representative energy trace demonstrating persistent inertial cavitation.

Inertial cavitation was also demonstrated using particles produced according to Example 20 above. Particles were exposed to 1.4 MPa, 1.7 MPa and 2.1 MPa for at least 300 seconds. Particles were studied both before and after freeze-drying. Water was used as a control. Cavitation was observed under all conditions for all particles. At 1.4 MPa, cavitation was observed for less than 20s for dried but non-freeze-dried particles, but for over 150s for freeze-dried particles. At 1.7 MPa, cavitation was observed for around 100s for dried but non-freeze-dried particles, but for over 150s for freeze-dried particles. At 2.1 MPa, cavitation was observed for around 200s for dried but non-freeze-dried particles, but for over 300s for freeze-dried particles.

### Effect of Percentage Volatile Component in Core on Cavitation Ability

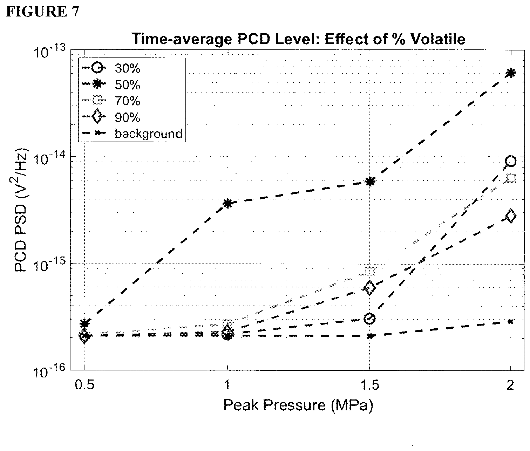

Examples 11 to 13 above describe the production of particles in an identical manner to Example 1 but using differing amounts of the volatile component, hexane. Experiments were conducted to establish if varying the ratio of volatile solvent (and as such the potential depth of the indentation in the particle) can affect the cavitation ability of the particle. Cavitation was studied in the same manner as set out above and the results are set out in Figure 7. Varying the volatile component (and thus the depth of the indentation) had a significant effect on the cavitation potential of the particles. 50% by volume volatile was optimum for cavitation of the resulting particle, although cavitation was detected at all volatile content levels.

### In Vivo Cavitation Ultrasound Methodology

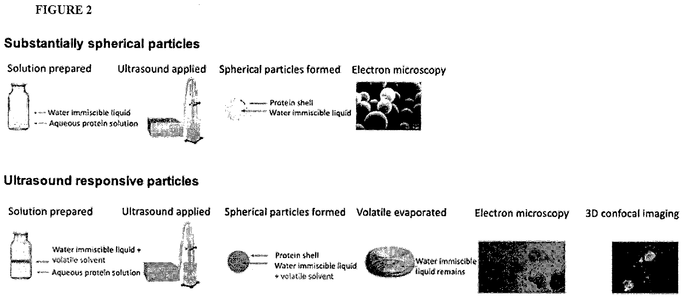

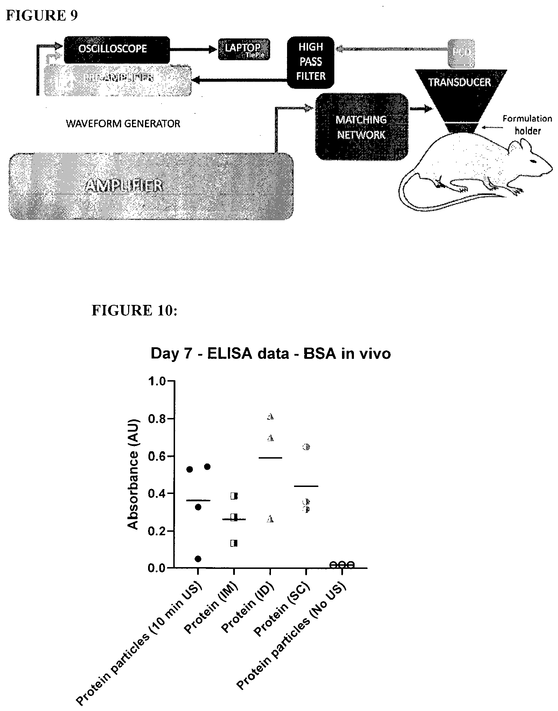

In vivo transdermal particle cavitation in the experiments described below was carried out by using a source transducer (117D-01, Sonic Concepts, USA) to initiate cavitation. The transducer was fitted with a Perspex coupling cone (used to efficiently transmit ultrasound from the source to the therapy target), filled with degassed water and covered with an acoustically transparent Mylar plastic. The coupling cone shape was designed to align with the outer envelope of the focused ultrasound beam. A formulation holder, containing particle solution, was placed on the murine skin and the source transducer was placed on top of this, with the Mylar plastic acoustic window positioned to prevent air entering the acoustic path. A single element spherically focused ultrasound transducer acting as passive acoustic detector (PCD) was coaxially located inside the source transducer, to allow the cavitation signal to be recorded. The PCD signal was passed through a 2.0 MHz high pass filter and amplified with a gain of 25 by a pre-amplifier (SR445A, Stanford Research Systems, USA). The signal was then digitised and monitored in real time by an 12-bit digital oscilloscope (Handyscope HS3 100 MHz, TiePie Engineering, The Netherlands) and the data was saved on an external hard drive (Portable SSD T5, Samsung, Korea) (Figure 2). This specific cavitation detection setup has been documented in Gray et al, IEEE Trans. UFFC 65(2) 2018, pp 258-267. The set-up is schematically depicted in Figure 9.

### Transdermal Particle Delivery and In Vivo Cavitation

Particles according to the invention were applied to mice to study the transdermal delivery of the particles. Immune response to the protein delivered was used to assess successful delivery of particle via transdermal administration followed by ultrasound.

8 mice were used in the experiment, and each mouse received two separate particle preparations, one applied to each flank. Thus, 16 experiments were carried out in total as follows:
Group 1: 4 x Protein particles + US
Group 2: 3 x Protein particles without US
Group 3: 3 x protein subcutaneous injection
Group 4: 3 x protein Intradermal injection
Group 5: 3 x protein Intramuscular injection

Protein particles used were particles produced in accordance with Example 1 above. The particles were administered to each mouse either by direct application to the mouse flank (with or without application of ultrasound), or by injection as indicated above. The total protein content of each component administered was 9mg. It is important to note that for injection, the full 9mg is delivered to the mouse, whereas for transdermal administration, only a fraction of the 9mg protein is delivered. Where ultrasound was used, this was carried out as discussed in *"In Vivo Cavitation Ultrasound Methodology"* above. Exposure time was 10 minutes.

Following administration of the particles in the designated manner for each group, immune response to BSA protein was determined by carrying out ELISA analysis to study immune response to BSA following delivery.

Results are depicted in Figure 10. Immune response to BSA in the transdermal group (Group 1) was comparable to that of the injection groups, showing successful delivery of BSA via the transdermal route.

### Transdermal delivery of DNA encoding luciferase via cavitation of protein particles

Particles according to the invention, or polymer particles produced in accordance with WO 2015/075422, were applied to a mouse flank. 8 mice were used in the experiment, and each mouse received two separate particle preparations, one applied to each flank. Thus, 16 experiments were carried out in total as follows:
- 4 x protein particles + DNA Luc (10 minute US exposure)
- 4 x protein particles + DNA Luc (2 minute US exposure)
- 4 x polymer particles + DNA Luc (10 minute US exposure)
- 4 x protein particles + DNA Luc (10 minute No US Sham)

Protein particles used were particles produced in accordance with Example 1 above. Polymer particles were produced in accordance with WO 2015/075422. All samples were provided in PBS. Protein particles were provided as 9mg protein in 1.8ml PBS. All samples contained 50µg of DNA encoding luciferase. The particles/DNA were administered to each mouse by direct application to the mouse flank (with or without application of ultrasound). Ultrasound (US) was applied using the method described in *"In Vivo Cavitation Ultrasound Methodology"* above, for the indicated exposure time.

Successful transdermal delivery was assessed by the generation of luciferase following administration. Figure 11 shows bio-luminescence measurements carried out via IVIS imaging at 22 hours post treatment. Results are compared with bioluminscence generated following direct injection (via intramuscular, intradermal or subcutaneous injection) of DNA encoding luciferase. Injected solutions were identical to the protein particle solutions used for transdermal administration.

Transdermal administration of DNA using the particles of the invention as cavitation delivery agent produced comparable or significantly greater luminescence in all cases when compared to current gold standard cavitation agent (polymer particles).

Particles according to Example 20, together with luciferase pDNA, were also administered transdermally by the same method. Figure 8A shows the energy detected by PCD over time at 1.7 MPa during in vivo transdermal delivery of luciferase pDNA. At three different timepoints the frequency domain is plotted to show that inertial cavitation was dominant. A 2 MHz high-pass filter was used in acquiring the acoustic emissions data from the passive cavitation detector, hence no signal was visible below 2MHz on the spectrograms.

## Claims

1. A polypeptide particle for inducing cavitation on exposure to ultrasound, the particle comprising:
- a core comprising one or more therapeutic or diagnostic agents, and optionally a water-immiscible liquid; and
- a polypeptide shell;
wherein the particle has one or more surface indentations.

2. A polypeptide particle according to claim 1, wherein the polypeptide shell comprises one or more non-immunomodulatory polypeptides, preferably the polypeptide shell comprises at least 95% by weight non-immunomodulatory polypeptides, more preferably wherein the polypeptide shell is a human serum albumin shell.

3. A polypeptide particle according to any one of the preceding claims, wherein the particle size is in the range of from 100nm to 1000nm, preferably from 200nm to 700nm.

4. A polypeptide particle according to any one of the preceding claims, wherein the cross section of at least one surface indentation forms a conic section; and/or wherein at least one surface indentation has a depth of at least 10nm; and/or wherein the particle comprises one or two indentations having an opening size which is 20% or more of the size of the particle.

5. A polypeptide particle according to any one of the preceding claims, which particle is capable of generating inertial cavitation when the particle is suspended in water and subject to ultrasound at 1.2MPa and 265kHz, preferably the particle is capable of generating inertial cavitation when the particle is suspended in water and subject to ultrasound at 1MPa and 500kHz, more preferably the particle is 450nm or less in size and is capable of generating inertial cavitation when the particle is suspended in water and subject to ultrasound at 1.5 MPa and 500kHz.

6. A polypeptide particle according to any one of the preceding claims, wherein the core comprises one or more therapeutic agents selected from small-molecule drugs, including chemotherapy agents, anti-thrombotic drugs, anti-inflammatory drugs, steroids, cardiovascular drugs, and therapeutic agents useful in the treatment of cancer, skin conditions, diseases or disorders of the brain, and therapeutic agents useful in pain management.

7. A polypeptide particle according to any one of the preceding claims, wherein the core is a water-immiscible liquid core comprising a water-immiscible liquid and one or more therapeutic or diagnostic agents.

8. A composition comprising one or more polypeptide particles according to any one of the preceding claims, together with one or more pharmaceutically acceptable carriers or diluents.

9. A method of producing a polypeptide particle according to any one of claims 1 to 7, comprising:
- providing a water-immiscible phase comprising one or more volatile components, one or more therapeutic or diagnostic agents, and optionally one or more non-volatile components, optionally wherein the ratio of volatile component to non-volatile component in the water-immiscible phase is from 1:20 to 20:1;
- mixing said water-immiscible phase with an aqueous solution of at least one polypeptide comprising at least two cysteine residues;
- cross-linking the cysteine residues to generate a particle having a core comprising the water-immiscible phase and a shell comprising the at least one polypeptide; and
- creating one or more indentations in the particle by subjecting the particle to reduced pressure conditions, to remove volatile component from the core; and optionally
- purifying the particle.

10. A method according to claim 9, which further comprises:
- drying the particle; and
- optionally re-suspending the particle in a liquid medium; and wherein the method may also further comprise:
- lyophilising the particle; and
- optionally re-suspending the particle in a liquid medium;
wherein preferably the lyophilisation and optional resuspension are carried out after drying the particle and re-suspending the particle in a liquid medium.

11. A polypeptide particle according to any one of claims 1 to 7, or a composition according to claim 8, for use in a method of diagnosis or therapy, preferably wherein the method comprises administering the polypeptide particle or composition, applying ultrasound and inducing inertial cavitation; optionally wherein the polypeptide particle or composition is administered by parenteral or transdermal administration.

12. A polypeptide particle or composition for use according to claim 11, wherein ultrasound is delivered at a frequency of from 100 to 3000kHz and a peak negative pressure of from 0.5 to 7.0 MPa; preferably wherein (a) the polypeptide particle or composition is administered transdermally and ultrasound is delivered at a frequency of from 100 to 500 kHz and a peak negative pressure of from 0.5 MPa to 2.0 MPa; or (b) the polypeptide particle or composition is administered by parenteral injection and ultrasound is delivered at a frequency of from 200 to 3000 kHz and a peak negative pressure of from 1.0 MPa to 7.0 MPa.

13. A polypeptide particle or composition for use according to any one of claims 11 to 13, wherein the method is for delivery of the therapeutic or diagnostic agent to a target site in vivo, preferably wherein the polypeptide particle or composition is for use in the delivery of the therapeutic or diagnostic agent to a tumour, to the heart, to the brain or to the skin.

14. A polypeptide particle or composition for use according to any one of claims 11 to 13, for use in treating or preventing tumour, cardiovascular disease, infection or a neurological disease or disorder.

15. A polypeptide particle or composition for use according to any one of claims 11 to 13, for use in treating or preventing a disease or condition of the skin.
